# EUROPEAN PATENT SPECIFICATION

(11) **EP 2 143 387 B1**
(45) Date of publication and mention of the grant of the patent: **21.12.2016**
(21) Application number: 09251756.4
(22) Date of filing: 08.07.2009
(51) Int. Cl.: A61B 17/11, A61F 2/06, A61B 17/00

(54) **Anastomosis sheath**
Anastomosehülle
Gaine d'anastomose

(30) Priority: 09.07.2008 US 79202 P; 17.06.2009 US 486352
(43) Date of publication of application: 13.01.2010
(73) Proprietor: Covidien LP, Mansfield, MA 02048 (US)
(72) Inventor: Stopek, Joshua, Yalesville, CT 06492 (US); Jones, Jacqueline, Hamden, CT 06518 (US); Elachchabi, Amin, Hamden, CT 06518 (US)
(74) Representative: Soames, Candida Jane

(56) References cited:
- EP-A- 1 702 571
- WO-A-2004/086984
- WO-A-2005/110280
- US-A- 4 182 339
- US-A- 5 151 105
- US-A1- 2005 273 060
- US-A1- 2008 039 878
- US-B1- 6 926 724

## Description

### Technical Field

The present disclosure relates to a sheath for use with an anastomosis for prevention of fluid leaks, and more particularly to a sheath which includes at least two ring-shaped members which retain the sheath in a body lumen so as to protect an anastomotic site.

### Background

An anastomosis, or the joining of two vessels, such as esophagus, colon, or other parts of the digestive tract, is a common procedure. Sometimes, however, there are complications associated with the anastomotic site.

Specific patient populations such as patients with diabetes T1, T2, or other immuno-compromised patients (such as chemotherapy patients) are more prone to anastomotic leaks. These patient populations have longer healing profiles and sometimes weaker immune systems and these factors may lead to an increase in leak occurrence. Unfortunately, in most cases, anastomotic leaks are not detected until clinical symptoms present themselves.

While current anastomotic devices and surgical methods perform satisfactorily, it would be advantageous to provide a device to reduce the risks associated with anastomotic leaks.

The two part form of claim 1 is based on US 6926724 B1.

### SUMMARY

The invention is described in claim 1, preferred embodiments are described in the dependent claims.

A sheath is described herein which provides for protection of an anastomosis and a reduction in anastomotic leaks. The sheath includes a sleeve defining a passage, a first ring-shaped member and a second ring-shaped member, wherein a proximal portion of the sleeve is connected to the first ring-shaped member and a distal portion of the sleeve is connected to the second ring-shaped member. The first ring-shaped member is positioned proximal of the anastomosis and the second ring-shaped member is positioned distal of the anastomosis. Preferably, the first ring-shaped member and the second ring-shaped member each apply a radial force to a body lumen wall and are in direct contact with a body lumen wall. The first and second ring-shaped members are preferably filled with a fluid or foam which may be thermo responsive or pressure sensitive.

The first ring-shaped member is disposed at an angle relative to a longitudinal axis of the sleeve so as to direct a flow of fluids away from a body lumen wall. Furthermore, in a preferred embodiment, at least a portion of the first ring-shaped member is generally curved to as to direct a flow of fluids away from a body lumen wall. The first ring-shaped member may be convex or concave with respect to a longitudinal axis of the sleeve.

In a preferred embodiment, the first and second ring-shaped members extend circumferentially around a periphery of the sleeve. In one embodiment, the first and second ring-shaped members comprise biodegradable materials.

In another aspect of the sheath described herein, the sheath includes a sleeve defining a passage, the sleeve having a proximal portion and a distal portion, and the sleeve including a first balloon and a second balloon. The proximal portion of the sleeve is connected to the first balloon and the distal portion of the sleeve is connected to the second balloon, wherein the first balloon is positioned proximal of the anastomosis and the first balloon has a radius of curvature which directs fluid flow of luminal contents away from a wall of the body lumen and into the passage of the sleeve, and the second balloon is positioned distal of the anastomosis.

In one aspect at least a portion of the first balloon is substantially concave or convex with respect to a longitudinal axis of the sleeve. The first balloon can be funnel-shaped. In some embodiments, a cross-sectional area of the first balloon is teardrop-shaped.

The first and second balloons preferably extend circumferentially around a periphery of the sleeve. Additionally, the first and second balloons, in one embodiment, comprise biodegradable materials. Preferably, the first balloon and the second balloon each apply a radial force to a body lumen wall and are in direct contact with a body lumen wall. Furthermore, in one embodiment, the first and second balloons are filled with a fluid or foam which may be thermo responsive or pressure sensitive.

The sleeve is configured for elongation longitudinally in situ from a first length to a second greater length. In certain embodiments, the sleeve further includes a grasping member at the proximal end to facilitate elongation of the sleeve.

Fluids for filling the ring shaped member or balloon may be selected, for example, from the group consisting of water, saline, buffers, wound healing agents, polymer solutions, gels, oxygen, nitrogen, carbon dioxide, and air. Sheaths of the present disclosure may further comprise a bioactive agent.

Methods for using the sheaths are also disclosed. In one aspect, the method comprises the steps of providing a sleeve defining a passage, the sleeve having a distal portion connected to a distal ring-shaped member and a proximal portion connected to a proximal ring-shaped member, attaching the proximal ring-shaped member to a body lumen at a region proximal of an anastomosis; and elongating the sleeve from a first length to a second length, the distal ring-shaped member of the elongated sleeve being positioned distal to the anastomosis.

### BRIEF DESCRIPTION OF THE DRAWINGS

The illustrative embodiments described herein will become more readily apparent from the following description, reference being made to the accompanying drawings in which:
FIG. 1 is a perspective view of one embodiment of a sheath in accordance with the present disclosure;
FIG. 2A is a perspective view of an alternate embodiment of a sheath in accordance with the present disclosure;
FIG. 2B is a partial, cross-sectional view of the embodiment of FIG. 2A in accordance with the present disclosure;
FIG. 3A is a perspective view of another embodiment of a sheath in accordance with the present disclosure;
FIG. 3B is a partial, cross-sectional view of the embodiment of FIG. 3A in accordance with the present disclosure;
FIG. 4A is a perspective view of one alternate embodiment of a sheath of the present disclosure in a first, compressed position;
FIG. 4B is a perspective view of the sheath of FIG. 4A in a second, elongated position;
FIG. 5A is a perspective view of the sheath of FIG. 1 showing the ring-shaped member in a first position; and,
FIG. 5B is a perspective view of the sheath of FIG. 1 showing the ring-shaped member in a second, radially expanded position.

### DETAILED DESCRIPTION OF EMBODIMENTS

The present disclosure is directed to an anastomotic sheath. The sheath includes a sleeve having a passage, which allows for the transport and flow of fluids therethrough. The sleeve is connected at a proximal end to a first ring-shaped member and the sleeve is connected at a distal end to a second ring-shaped member. In certain embodiments, the ring-shaped members are balloons such that the sleeve is connected at the proximal end to a first balloon and the sleeve is connected at the distal end to a second balloon. In some embodiments, the first balloon directs a fluid towards a passage defined by the sleeve. The balloons and ring-shaped members may be filled with fluids or foams. Additionally, the sleeves are configured for elongation from a first length to a second longer length. Sheaths of the present disclosure may be made from a variety of materials including both biodegradable and non-biodegradable materials.

In the description that follows, the term "body lumen" as used herein, means inner open space or cavity of a tubular organ, such as a blood vessel, intestine, or esophagus. The term "biodegradable" as used herein refers to materials which decompose, or lose structural integrity under body conditions (e.g., enzymatic degradation or hydrolysis). The term "proximal" as used herein, means the portion of the sheath which is nearer to the user, while the term "distal" refers to the portion of the sheath which is further away from the user.

The sheath, at least in part, may be comprised of biodegradable materials which include both synthetic and natural materials. Suitable synthetic biodegradable materials include polymers such as those made from lactide, glycolide, caprolactone, valerolactone, carbonates (e.g., trimethylene carbonate, tetramethylene carbonate, and the like), dioxanones (e.g., 1,4-dioxanone), δ-valerolactone, 1,dioxepanones (e.g., 1,4-dioxepan-2-one and 1,5-dioxepan-2-one), ethylene glycol, ethylene oxide, esteramides, γ-hydroxyvalerate, β-hydroxypropionate, alpha-hydroxy acid, hydroxybuterates, poly (ortho esters), hydroxy alkanoates, tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof. Suitable natural biodegradable polymers include collagen, cellulose, poly (amino acids), polysaccharides, hyaluronic acid, gut, copolymers and combinations thereof.

Suitable non-biodegradable materials which may be used to construct the sheath include fluorinated polymers (e.g.,fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, copolymers and combinations thereof. Additionally, non-biodegradable polymers and monomers may be combined with each other and may also be combined with various biodegradable polymers and monomers to create a composite sheath.

In certain embodiments, sheaths according to the present disclosure may be constructed at least in part using shape memory polymers. Suitable polymers used to prepare hard and soft segments of shape memory polymers include polycaprolactone, dioxanone, lactide, glycolide, polyacrylates, polyamides, polysiloxanes, polyurethanes, polyether amides, polyurethane/ureas, polyether esters, and urethane/butadiene copolymers and combinations thereof.

In some embodiments, the sheath may comprise metals (e.g., steel or titanium), metal alloys and the like. In alternate embodiments, a grasping structure may comprise degradable metals such as degradable magnesium.

Suitable materials of the present disclosure can be processed within the purview of those skilled in the art including, but not limited to extrusion, injection molding, compression molding, blow molding, film blowing, thermoforming, calendaring, spinning, and film casting.

The sheath preferably includes a sleeve which elongates and extends distally past an anastomotic site. In some embodiments, the sleeve is film which may be porous or semi-permeable to enable or restrict oxygen and nutrient transport. Alternatively, semi-permeable or controlled permeability properties along some or all of the sleeve's length allow absorption of certain nutrients at the appropriate location in the body lumen wall. For example, when the sheath is used in the intestines, nutrient absorption at a specific location along the gastrointestinal tract is desirable to avoid malabsorption. Alternately, a specific length or the entire length of the sleeve may be non-porous or impermeable. It will be understood that other embodiments are within the purview of those skilled in the art and are within the context of the present disclosure. For example, alternate embodiments such as foams or woven fibers may be preferred to films when the sleeve's surface area is used to alter the degradation times and profiles. It should also be understood that the above discussion including a structure's permeability, for example, a semi-permeable membrane or controlled permeability, are not limited to a sleeve and may also include additional parts of the sheath (such as a balloon). Films of the present disclosure may be manufactured using methods within the purview of those skilled in the art including, but not limited to the methods listed above.

Optionally the sheath can include coatings on its interior and/or exterior to enhance the surface properties of the sheath in clinically relevant manners. As used herein, the term "coating" is not limited to liquids and may also include waxes and solids. For example, a parylene coating may be used to increase the chemical resistance of the sleeve material. In other embodiments, lubricious coatings which aid in nutrient passage through sheath such as poly ethylene glycols may be applied to the sheath. Coatings may be applied using any method within the purview of those skilled in the art.

Additionally, any part of the sheath may include biologically acceptable additives such as plasticizers, antioxidants, dyes, image-enhancing agents, dilutants, bioactive agents such as pharmaceutical and medicinal agents, and combinations thereof which can be coated on the sheath or impregnated within the resin or polymer.

Medicinal agents which may be incorporated into the sheath include antimicrobial agents, anti-virals, anti-fungals, and the like. Antimicrobial agents, as used herein, is defined as an agent which, by itself or through assisting the body (immune system), helps the body destroy or resist microorganisms which may be pathogenic (disease causing). The term "antimicrobial agent" includes antibiotics, quorum sensing blockers, surfactants, metal ions, antimicrobial proteins and peptides, antimicrobial polysaccharides, antiseptics, disinfectants, anti-virals, anti-fungals, quorum sensing blockers, and combinations thereof. Examples of suitable antiseptics and disinfectants that can be combined with the present disclosure include hexachlorophene, cationic biguanides like chlorhexadine and cyclohexidine, iodine and iodophores like povidone-iodine, halo-substituted phenolic compounds like PCMX (e.g., p-chloro-m-xylenon) and triclosan (e.g., 2,4,4'-trichloro-2'hydroxy-diphenylether), furan medical preparations like nitrofurantoin and nitrofurazone, methanamine, aldehydes like gluteraldehyde and formaldehyde, alcohols, combinations thereof, and the like.

Classes of antibiotics that can be combined with the present disclosure include tetracyclines like minocycline, rifamycins like rifampin, macrolides like erythromycin, penicillins like nafcillin, cephalosporins like cefazolon, beta-lactam antibiotics like imipenen and aztreonam, aminoglycosides like gentamicin and TOBRAMYCIN®, chloramphenicol, sulfonamides like sulfamethoxazole, glycopeptides like vancomycin, quilones like ciproflaxin, fusidic acid, trimethoprim, metronidazole, clindamycin, mupirocin, polyenes like amphotericin B, azoles like fluconazole, and beta-lactam inhibitors like sublactam. Other antimicrobials which may be added include, for example antimicrobial peptides and/or proteins, antimicrobial polysaccharides, quorum sensing blockers (e.g., brominated furanones), anti-virals, metal ions such as ionic silver and ionic silver glass, surfactants, chemotherapeutic drug, telomerase inhibitors, other cyclic monomers including 5-cyclic monomers, mitoxantrone, and the like.

In some embodiments, suitable bioactive agents which may be used include colorants, dyes, preservatives, protein and peptide preparations, protein therapeutics, polysaccharides such as hyaluronic acid, lectins, lipids, probiotics, angiogenic agents, anti-thrombotics, anti-clotting agents, clotting agents, analgesics, anesthetics, wound repair agents, chemotherapeutics, biologics, anti-inflammatory agents, anti-proliferatives, diagnostic agents, antipyretic, antiphlogistic and analgesic agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, antiviral agents, dysuric agents, brominated or halogenated furanones, and the like. In embodiments, polymer drugs, e.g., polymeric forms of compounds such as, polymeric antibiotics, polymeric antiseptics, polymeric chemotherapeutics, polymeric anti-proliferatives, polymeric antiseptics, polymeric non-steroidal anti-inflammatory drugs (NSAIDS), and the like may be utilized and combinations thereof.

In certain embodiments, sheaths of the present disclosure may contain suitable medicinal agents such as viruses and cells, peptides, polypeptides and proteins, analogs, muteins, and active fragments thereof, such as immunoglobulins, antibodies (monoclonal and polyclonal), cytokines (e.g., lymphokines, monokines, chemokines), blood clotting factors, hemopoietic factors, interleukins (IL-2, IL-3, IL-4, IL-6), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.) hormones and hormone analogs (e.g., growth hormone), vaccines (e.g., tumoral, bacterial and viral antigens), somatostatin, antigens, blood coagulation factors, growth factors, protein inhibitors, protein antagonists, and protein agonists, nucleic acids, such as antisense molecules, DNA, RNA, oligonucleotides, polynucleotides and ribozymes and combinations thereof.

In some embodiments, additives such as image-enhancing agents (e.g., contrast agents) and more specifically, radiopaque markers, may be incorporated into the sheath. These image-enhancing agents enable visualization of the sheath (against surrounding tissue) when imaged or scanned through different filters such as MRI, X-ray, fluoroscopy, CT, various light sources, and the like. In order to be opaque (and visualized in certain filters), the sheath must be made from a material possessing a radiographic density higher than the surrounding host tissue and have sufficient thickness to affect the transmission of x-rays to produce contrast in the image. Useful image-enhancing agents include but are not limited to radiopaque markers such as tantalum, barium sulfate, bismuth trioxide, bromine, iodide, titanium oxide, zirconium, barium, titanium, bismuth, iodine, nickel, iron, silver, and combinations thereof. In some embodiments, compounds such as tantalum, platinum, barium and bismuth may be incorporated into the sheath. Often image-enhancing agents are not bioabsorbable or degradable but are excreted from the body or stored in the body.

Image-enhancing agents may be compounded into the materials (e.g., resin) as filler prior to processing including extrusion or molding. These agents may be added in various concentrations to maximize polymer processing while maximizing the material characteristics of the sheath. The biocompatible agents can be added in quantities sufficient to enhance radiopacity while maintaining the polymer's properties. In certain embodiments, image-enhancing agents may be incorporated into a biodegradable portion, enabling surgeons to know when the biodegradable portion has degraded.

Methods for combining the above mentioned bioactive agents with materials of the present disclosure are within the purview of those skilled in the art and include, but are not limited to mixing, blending, compounding, spraying, wicking, solvent evaporating, dipping, brushing, vapor deposition, coextrusion, capillary wicking, film casting, molding and the like. Additionally, solvents may be used to incorporate various agents into the composite device. Suitable solvents include those listed above.

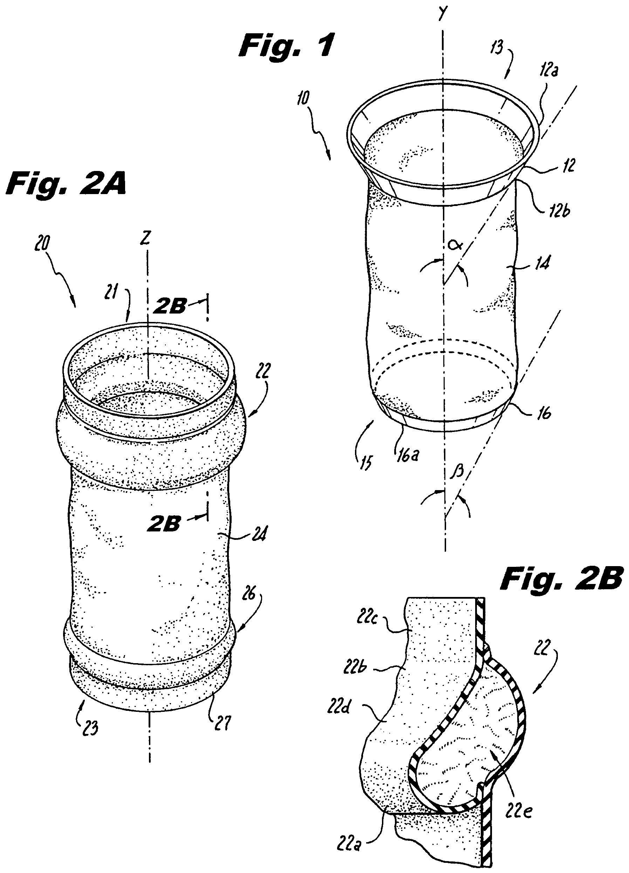

A first embodiment of an anastomotic sheath of the present disclosure is illustrated in Figure 1. The sheath 10 includes a sleeve 14 which is connected at a proximal portion 13 to a first ring-shaped member 12. The first ring-shaped member 12 extends circumferentially around the proximal portion 13 of the sleeve 14. The ring-shaped member 12 is generally funnel-shaped, and has as a larger diameter at a proximal end 12a, promoting a flow of luminal contents towards a distal opening 16a. The diameter of the first ring-shaped member 12 decreases towards a distal portion 12b. An inner wall of the first ring-shaped member 12 is disposed at an angle relative to a longitudinal axis "Y" of the sleeve 14 so as to better direct the passage of fluids through a distal opening 16a of the sheath 10. The relative angle α of the first ring-shaped member 12 may range from about 1° to about 90 °, although other angles are also contemplated. The proximal end 12a of the ring-shaped member 12 is in contact with a body lumen wall proximal of the anastomotic site so as to direct the flow of fluids away from the body lumen wall and towards a passage defined by the sleeve 14. In other embodiments, the first ring-shaped member 12 may be disposed at a series of angles or a series of curves so as to direct fluid flow away from the body lumen wall and towards a distal opening 16a of the sleeve 14, protecting the anastomotic site from luminal contents.

The sleeve 14 is connected at a distal portion 15 to a second ring-shaped member 16, which extends circumferentially around the distal portion 15 of the sleeve 14. The second ring-shaped member 16 is also in contact with a body lumen wall so as to prevent fluid from passing (during peristalsis) along a periphery of the sleeve 14 and contacting an anastomotic site. In some embodiments, the second ring-shaped member 16 may be disposed at an angle β relative to the longitudinal axis "Y" of the sleeve to help direct fluids towards distal opening 16a. Alternatively, the second ring-shaped member 16 may be disposed at a series of angles or a series of curves relative to the longitudinal axis "Y."

In some embodiments, the first ring-shaped member 12 has an internal space, defined between its external walls, and is preferably filled with a fluid or foam, which may assist in retaining the sheath 10 in the body lumen by applying a radial force against the body lumen. The second ring-shaped member 16 may also be filled with a fluid or foam, to secure the sheath by applying a radial force against the body lumen. The term "fluid" as used herein includes liquids, gels, and gasses. Examples of fluids which may be used include, but are not limited to, gasses such as oxygen, nitrogen, carbon dioxide, and liquids such as water, saline, buffers, solutions including polymer solutions, gels and in situ forming materials. Fluids and foams of the present disclosure may initially be in the form of dried solids (e.g. lyophilized particulates) and upon activation may gel or aerosolize. The disclosure contemplates both single component systems and multi-component systems which can be activated in situ by using external initiators/factors such as heat, energy, water, pressure, and the like. Additionally, the volume of fluid may be adjusted to apply a specific amount of pressure to a body lumen wall. For example, in patients whose tissue is weaker or less compliant, less radial force (on exerted on tissue) may be desired by the surgeon. In some embodiments, a valve (not shown) may be present on the sheath which enables fluid or foam volume adjustment prior to insertion or in vivo.

Figure 2A illustrates a sheath 20 in which each ring-shaped member 22, 26 is a balloon. The sheath 20 includes a first balloon 22 connected to a proximal portion 21 of a sleeve 24 and a second balloon 26 connected to a distal portion 23 of the sleeve 24. Each of the first and second balloons (22 and 26) extends circumferentially around a periphery of the sleeve 24. The cross-sectional area of the first balloon 22 is generally teardrop-shaped (FIG 2B), although other shapes are contemplated. The distal end 22a of the first balloon 22 is generally curved or semi-circular, forming the bottom of the teardrop. The proximal portion 22b of the first balloon 22 defines a tip or apex. Extending beyond the proximal portion 22b is a flat section 22c which may be used when additional fixation devices, such as sutures, staples and the like, are preferred. The flat section 22c may be used to suture, tack, or glue the sheath in a body lumen, decreasing the risk of puncturing the proximal balloon 22. In alternate embodiments, where additional support is not required, a flat section may not be present.

The first balloon 22 is in direct contact with a body lumen wall proximal to the anastomotic site, and once radially expanded, the first balloon 22 is positioned flush against the body lumen wall to direct the flow of fluids through the sleeve, enabling the luminal contents to bypass the anastomotic site. The first balloon 22 applies a radial force against the body lumen wall to help retain the sheath. An inner segment 22d of the first balloon 22 has a radius of curvature which directs fluid flow away from the body lumen wall and towards the sleeve 24. Inner segment 22d may be substantially concave with respect to a longitudinal axis "Z" of the sleeve 24. Luminal contents pass through a distal opening 27 of the sheath 20, bypassing the anastomotic site and reducing the risk for anastomotic leaks and/or limiting nutrient adsorption.

A second balloon 26 is connected to a distal portion of the sleeve 24. As illustrated in Figure 2A, the sleeve 24 extends beyond the second balloon 26, although in certain embodiments, the sleeve 24 does not extend beyond the second balloon 26. Upon radial expansion, the second balloon 26 may be in direct contact with a body lumen wall distal of the anastomotic site to prevent fluid from passing along a periphery of the sleeve 24 and contacting the anastomotic site. The second balloon 26 applies a radial force against the body lumen wall to help retain the sheath. The second balloon 26 may be curved or angled with respect to a longitudinal axis "Z" of the sleeve 24. The second balloon 26 may also be of a similar shape to the first balloon 22 or can be of a different cross-sectional shape than the first balloon 22.

In certain embodiments, an inner space 22e (FIG. 2E) of at least the first teardrop-shaped balloon 22 is filled with fluids or foams, and in preferred embodiments, both balloons 22, 26 are filled with one or more fluids and/or foams. It is also contemplated that only the second balloon is filled. The one or more fluids and/or foams radially expand the balloons 22, 26, providing mechanical support when mounting the sheath 20 in a body lumen. In an expanded position, the first and second balloons 22, 26 are flush against the body lumen wall and enable fluids to bypass the anastomotic site, reducing the potential for anastomotic leaks.

Balloons may be filled with thermo responsive (heat sensitive/heat activated) materials which are activated by body heat or upon reaching body temperature. Upon activation, the polymers may gel, harden, or otherwise radially expanding the balloon, securing the sheath in the body lumen. Thermo responsive systems may include single component or multi-component systems, which are activated upon the mixing of at least two components. For example, a single component system may comprise at least one component contained in the balloon, the component being activated upon implantation in a body lumen. An example of a multi component system may include a balloon containing a first component, where upon implantation, a second component is injected into the balloon (optionally through use of a port). Upon reaching body temperature, the mixture of the two components gel and radially expand the balloon against a body lumen wall.

Suitable materials for use in foam/gel systems which are thermo responsive include, but are not limited to, materials including poloxamers such as Pluronic® F68 and F-127 (PEG polypropylene glycol block copolymers), polyethylene glycol (PEG), poly lactide-co-glycolide (PLGA), poly caprolactone polymers and copolymers, chitosan and chitosan glycerol phosphate mixtures, hyaluronic acid, N-isopropylacrylamide (NIPAM), and combinations thereof. It should be understood that foams and fluids are used to fill balloons by way of example, and foams and fluids can be used in other embodiments described herein (such as ring-shaped members).

Alternate embodiments of multi-component systems are contemplated for use in an anastomotic sheath. For example, a balloon may contain a thin membrane which keeps at least two components separate and upon mixing the two components are activated and may gel or polymerize, or expand to provide mechanical support to the sheath. In some embodiments, a first component may be contained in the balloon and a second component may be injected prior to insertion of the sheath, alternately, the second component may be injected in situ. For example, one component may be prefilled in the balloon and a second component may be injected prior to implantation, the system reaching a final state (e.g., gel, foam, or solid) upon implantation.

Two component systems may react from about 1 second to about 60 minutes, in preferred embodiments, from about 1 second to about 30 minutes, providing mechanical support, applying radial pressure to a body lumen wall, fixating the sheath in place. Suitable polymers for use in multi-component systems include but are not limited to hyaluronic acid, alginate, chitosan, polylysine, polyvinyl pyrrolidone (PVP), polyhydroxyethyl methacrylate (PHEMA), polyacrylic acid (PAA), polymethacrylic acid (PMAA), polymethylmethacrylate (PMMA), polybutyl acrylate (PBA), polyurethanes, silanes and silicones, light reactive vinyl monomers and polymers, PEG N-hydroxysuccinimide ester (NHS), fibrin, gelatin, collagen, albumin, and synthetic proteins. Additionally, suitable compositions may contain salts such as multivalent ion salts (Ca, Mg, Gd, Fe, Cr, Al, phosphates, carbonates) or other primary or secondary amines for crosslinkers. It is also contemplated that crosslinkers or crosslinking solutions may be used as a second component to activate a two component system. Suitable crosslinkers include genipin, aldehydes, isocyanates, vinyl crosslinkers, silane coupling agents, or peptides.

Additionally, the volume of fluid may be adjusted to apply a specific amount of pressure to a body lumen wall. For example, a surgeon may desire that less radial force be exerted on the tissue in patients whose tissue is weaker or less compliant. In some embodiments, a valve may be present on the sheath to enable volume adjustment (of the foam/fluid) either prior to insertion, or in vivo. Alternatively, the balloon includes a self-sealing material, whereby an insertion point created by another device (e.g., syringe used to inject fluids into the balloon) will seal up upon removal of the device from the balloon, retaining injected fluids inside the balloon.

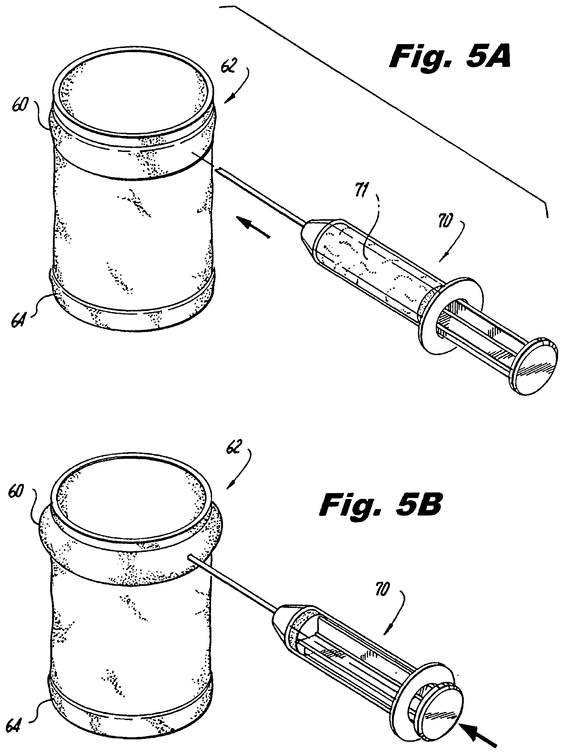

FIG. 5A illustrates a sheath 60 having compressed balloons 62, 64 by way of example, and not limitation, it being understood that other sheaths disclosed herein can be similarly inserted in a compressed configuration and filled with one or more fluids and/or foams 71 (as described above) which expand the balloons 62, 64 radially to a second position. A syringe 70 may inject one or more fluids and/or foams 71 into one or both of the balloons 62, 64. As shown in FIG. 5B, the material may expand one or both of the balloons 62, 64 to a second, radially expanded position (see, e.g. balloon 62). Other devices, such as catheters, may be used to fill/inject a balloon with one or more fluids and/or foams.

In certain preferred embodiments, biodegradable and biocompatible polymers are used to fill the balloons. It should also be understood that although a teardrop-shaped balloon is described for including both fluid and foam filled embodiments, other cross-sectional shapes of balloons and ring-shaped members can be provided and filled with fluids and foams and that the shapes described herein are non-limiting.

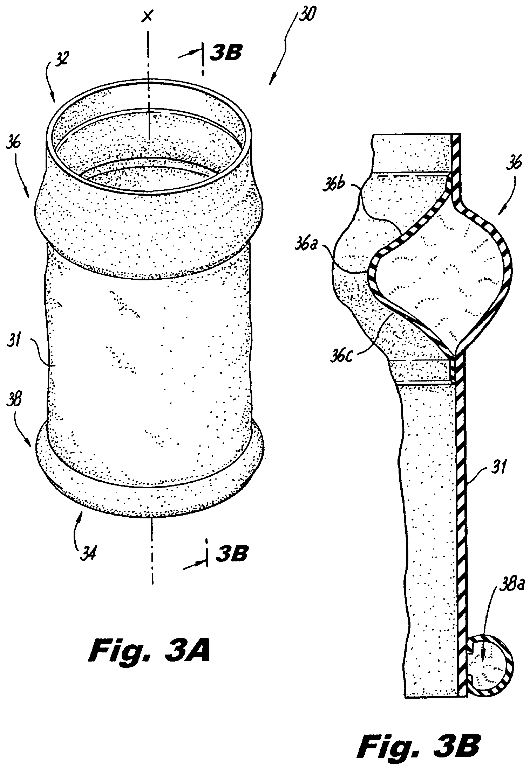

Another embodiment of a sheath is illustrated in FIG. 3A. An anastomotic sheath 30 has a sleeve 31 including a first balloon 36 at a proximal end 32 thereof, and a second balloon 38 connected to a distal end 34 of the sleeve 31. Each of the first and second balloons 36, 38 may be disposed in direct contact with a body lumen wall while extending circumferentially around a periphery of the sleeve 31. The first balloon 36 is generally semi-circular in cross-sectional area, while the second balloon 38 is generally elliptical in cross-sectional area. The present disclosure is not limited to the above described shapes and it should be recognized that other shapes are envisioned for the first and second balloons 36, 38 including oval, circular and polygonal.

Referring now to FIG. 3B, the first balloon 36 includes an inner segment 36b which is generally concave with respect to a longitudinal axis "X" of the sleeve 31. The inner segment 36b has a radius of curvature which directs fluid flow away from the body lumen wall and towards the interior of the sleeve 31. An inner distal end 36c of the first balloon 36 is curved so as to promote fluid flow towards a passage defined by the sleeve 31. The inner distal end 36c is shown as generally concave with respect to a longitudinal axis "X." In other embodiments, the inner distal end 36c may be generally convex with respect to the longitudinal axis "X" of the sleeve 31. Furthermore, an inner portion of at least the first balloon 36 may include one or more inflection points relative to the longitudinal axis "X" of the sheath 31. The first balloon 36 defines a hollow space 36a. The second balloon 38 also includes a hollow space 38a. In certain preferred embodiments, the hollow spaces 36a or 36b are filled with fluids, such as air or liquids, or foams, which expand the balloons 36, 38 and provide mechanical support to the sheath 30 in the same manner as described above. Suitable materials for use in filling the balloons 36, 38 include those listed above.

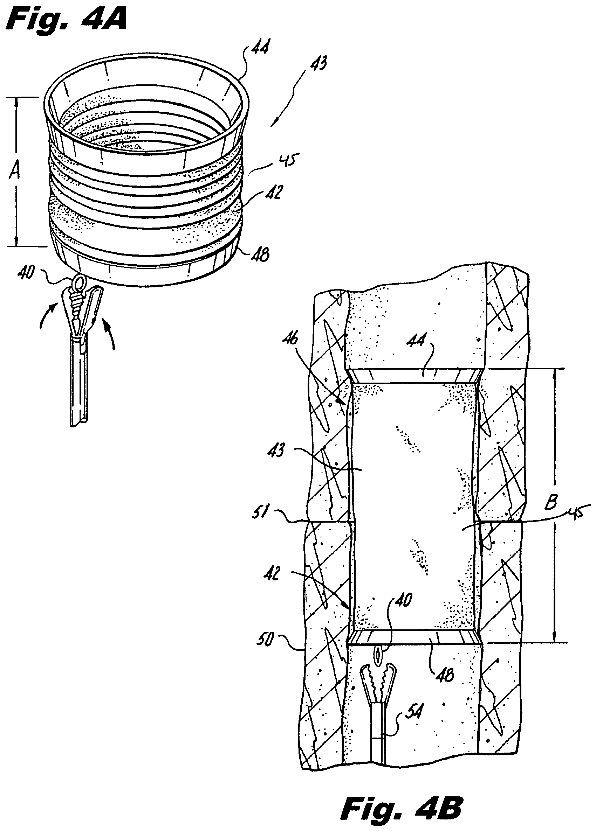

Another embodiment of a sheath is shown for example in FIG. 4A. The sheath 43 is generally similar to the embodiment described in FIG. 1, but further includes a grasping structure 40. The grasping structure 40 is a ring that is connected to a distal end 42 of the sheath 43. The grasping structure 40 may be a mesh, disc, O-ring, or of any shape which defines an opening (e.g. ring). Suitable materials for the grasping structure 40 include rigid (as compared to the sleeve 45) and flexible materials which may be either biodegradable or non-biodegradable materials such as those metals and polymers listed above. It should be noted that the various grasping structures disclosed may be used with any of the disclosed sheath embodiments.

The sheaths described herein are preferably movable from a collapsed, reduced length position to and extended, increased length position. The grasping structure facilitates such elongation as described below. More specifically, the sheath is preferably inserted into a body lumen in a compressed configuration, having for example a first length A, and then once in a body lumen, elongated to a second position having for example a second length B.

FIG. 4A illustrates the sheath 43 in a compressed configuration and having a longitudinal length A, by way of example, it being understood that other sheaths disclosed herein can be similarly inserted in a compressed configuration and extended to an elongated configuration. The grasping structure 40 facilitates elongation of the sheath 43 via the sleeve 45. The sheath 43 is preferably inserted in a compressed configuration and then once in a body lumen, elongated to a second position. As shown in FIG. 4B, once inserted into a body lumen 50, the ring-shaped member 40 applies a radial force to the body lumen 50, mounting the proximal end 46 of the sheath 43 in place. The grasping structure 40 may then be grasped by a surgical device 54, such as a grasper, which applies a downward force to the grasping structure 40 (as viewed in the orientation of FIG. 4B) and pulls the sleeve 45 distally, elongating the length of the sheath 43 to a length B, and extending the coverage of sheath 43 across the anastomotic site 51. A second ring-shaped member 48 also provides a radial force to the body lumen 50, preventing any backflow of luminal contents, reducing the potential for anastomotic leaks. The elongated sheath 43 enables fluids to bypass the anastomosis, reducing the amount of fluid contact with the anastomotic site and therefore reducing the potential for anastomotic leaks into the surrounding environment. It should be noted that the sheath 43 can be mounted to the body lumen wall 50 proximal to an anastomosis site before, during, or after anastomosis creation. After a length of time, the sheath may be excreted through the body via natural passageways or alternatively, the sheath may be surgically removed.

Additionally, the grasping structure 40 may further assist the user in positioning the sheath 43 in the body lumen 50 after elongation, without damaging the sleeve 45. The surgeon may grasp the grasping structure 40 and distally pull the grasping structure 40 with enough force to move the sheath 43 distally, repositioning the sheath 43 in the body lumen 50. Alternatively, an inverted sheath 43 may be inserted into the body lumen 50, attached to the body lumen 50, and then extended through the body lumen 50, distal to the anastomotic site.

In preferred embodiments, the sheath is generally tubular in shape and extends along a longitudinal axis of a body lumen. Other shapes are envisioned including, but not limited to, elliptical, conical, polygonal, and rectangular. The shape and concavity of the sheaths of the present disclosure may vary depending on factors such as the method of use and patient anatomy.

When the sheath is used in the intestinal tract, it is desirable to have the sheath flexible enough so as to allow the peristaltic motions of the intestines to effect movement of food through the composite sheath. There should, however, be enough friction between the sheath and gastrointestinal tract so that peristalsis will act to straighten the sheath and apply a small amount of tension to keep the sheath in place.

Preferably, the sheath has a balance of mechanical properties such that the sheath maintains coverage over the anastomosis in a longitudinally expanded position. The sheath preferably maintains enough rigidity to prevent the sheath from retracting proximally or folding on itself, which may expose the anastomosis site. The surface of the sheath may be configured with small bumps or other surface features which will enhance the friction between the sheath and the body lumen.

In certain embodiments, the fluids and foams may contain bioactive agents which may be released when the balloon or ring-shaped member degrades. Agents may diffuse through a balloon or a ring-shaped member into the adjacent tissue area or sheath interior. Other methods for the release of bioactive agents from the balloons and ring-shaped members are contemplated such as leaching and controlled release methods including diffusion. Drugs and bioactive agents may also be further protected or encapsulated for controlled release by the use of micro or nanoparticles. The sheath may be chemically altered to enable a specific drug or bioactive agent release profile.

Various degradation profiles and times are contemplated for any biodegradable portion of the sheath. For example, in one embodiment, the sleeve is comprised of a biodegradable material which may have a persistence time of 1 day to 12 weeks, in more preferred embodiments, 3 days to 21 days. The mass loss can be tailored to correspond closely to the strength loss of the biodegradable portion, hence when the sheath loses the mechanical properties of the biodegradable portion, the mass of material remaining will be dimished, so such as to mitigate inflammation and encapsulation.

It should be noted that the present disclosure is not limited to use with colonic and intestinal anastomoses and contemplates use at other anastomotic sites such as vascular anastomoses. Additionally, the above description contains many specifics; these specifics should not be construed as limitations on the scope of the disclosure herein but merely as exemplifications of particularly useful embodiments thereof. Those skilled in the art will envision many other possibilities within the scope of the disclosure as defined by the claims appended hereto.

## Claims

1. A sheath (10) for positioning within a body lumen adjacent an anastomosis for the treatment of fluid leakage, the sheath comprising:
a sleeve (14) defining a passage, the sleeve having a proximal portion (13) and a distal portion (15);
a first ring-shaped member (12); and
a second ring-shaped member (16);
wherein the proximal portion of the sleeve (13) is connected to the first ring-shaped member (12) and the distal portion of the sleeve (15) is connected to the second ring-shaped member (16), the first ring-shaped member (12) being positioned proximal of the anastomosis, and the second ring-shaped member (16) being positioned distal of the anastomosis,
when in use; and
wherein the sleeve (10) is in a compressible configuration such that it is capable of elongation longitudinally in situ from a first length to a second greater length, **characterised in that** the first ring-shaped member (12) is funnel-shaped, and has a larger diameter at a proximal end (12a) wherein the diameter of said first ring-shaped member (12) decreases towards a distal portion (12b).

2. The sheath (10) of claim 1, wherein the first (12) and second (16) ring-shaped members are filled with at least one fluid or foam.

3. The sheath (10) of claim 2, wherein the foam is a pressure sensitive foam.

4. The sheath (10) of claim 2, wherein the foam is a thermo responsive foam.

5. The sheath (10) of claim 2, wherein the fluid is a pressure sensitive fluid.

6. The sheath (10) of claim 2, wherein the fluid is a thermo responsive fluid.

7. The sheath (10) of claim 1, wherein the first (12) and second (16) ring-shaped members extend circumferentially around a periphery of the sleeve (14).

8. The sheath (10) of claim 1, wherein the first (12) and second (16) ring-shaped members comprise biodegradable materials.

9. The sheath (10) of claim 1, further comprising a bioactive agent.

10. The sheath (10) of claim 1, wherein the sleeve (14) further includes a grasping member (40) at the proximal end (13) to facilitate elongation of the sleeve.

11. A sheath (10) according to any preceding claim, wherein at least one ring member (12, 16) is a first balloon and at least one ring member is a second balloon.

## Patentansprüche

1. Umhüllung (10) für das Positionieren innerhalb eines Körperlumens, benachbart zu einer Anastomose, zur Behandlung des Austretens eines Fluids, wobei die Umhüllung umfasst:
einen Mantel (14), der einen Durchgang definiert, wobei der Mantel einen proximalen Abschnitt (13) und einen distalen Abschnitt (15) aufweist;
ein erstes ringförmiges Element (12); und
ein zweites ringförmiges Element (16);
wobei der proximale Abschnitt des Mantels (13) mit dem ersten ringförmigen Element (12) verbunden ist und der distale Abschnitt des Mantels (15) mit dem zweiten ringförmigen Element (16) verbunden ist, wobei, bei der Anwendung, das erste ringförmige Element (12) proximal von der Anastomose positioniert ist und das zweite ringförmige Element (16) distal von der Anastomose positioniert ist; und
wobei der Mantel (10) derart in einer zusammendrückbaren Konfiguration ist, dass er in situ zur Verlängerung in Längsrichtung von einer ersten Länge zu einer zweiten größeren Länge in der Lage ist, **dadurch gekennzeichnet, dass** das erste ringförmige Element (12) trichterförmig ist und einen größeren Durchmesser an einem proximalen Ende (12a) aufweist, wobei der Durchmesser des ersten ringförmigen Elements (12) in Richtung eines distalen Abschnitts (12b) abnimmt.

2. Umhüllung (10) nach Anspruch 1, wobei die ersten (12) und zweiten (16) ringförmigen Elemente mit mindestens einer Flüssigkeit oder einem Schaum gefüllt sind.

3. Umhüllung (10) nach Anspruch 2, wobei der Schaum ein druckempfindlicher Schaum ist.

4. Umhüllung (10) nach Anspruch 2, wobei der Schaum ein thermoresponsiver Schaum ist.

5. Umhüllung (10) nach Anspruch 2, wobei das Fluid ein druckempfindliches Fluid ist.

6. Umhüllung (10) nach Anspruch 2, wobei das Fluid ein thermoresponsives Fluid ist.

7. Umhüllung (10) nach Anspruch 1, wobei die ersten (12) und zweiten (16) ringförmigen Elemente sich in Umfangsrichtung rund um einen Umfang des Mantels (14) erstrecken.

8. Umhüllung (10) nach Anspruch 1, wobei die ersten (12) und zweiten (16) ringförmigen Elemente biologisch abbaubare Materialien aufweisen.

9. Umhüllung (10) nach Anspruch 1, die ferner ein biologisch aktives Mittel umfasst.

10. Umhüllung (10) nach Anspruch 1, wobei der Mantel (14) ferner ein Greifelement (40) an dem proximalen Ende (13) umfasst, um die Längenausdehnung des Mantels zu erleichtern.

11. Umhüllung (10) gemäß einem der vorhergehenden Ansprüche, wobei mindestens ein Ringelement (12, 16) ein erster Ballon ist und mindestens ein Ringelement ein zweiter Ballon ist.

## Revendications

1. Gaine (10) à positionner à l'intérieur d'un conduit corporel adjacent à une anastomose pour le traitement de fuite de fluides, la gaine comprenant :
un manchon (14) définissant un passage, le manchon ayant une partie proximale (13) et une partie distale (15) ;
un premier élément annulaire (12) ; et
un second élément annulaire (16) ;
dans laquelle la partie proximale du manchon (13) est reliée au premier élément annulaire (12) et la partie distale du manchon (15) est reliée au second élément annulaire (16), le premier élément annulaire (12) étant positionné de façon proximale à l'anastomose et le second élément annulaire (16) étant positionné de façon distale à l'anastomose,
lors de l'utilisation ; et
dans laquelle le manchon (10) est dans une configuration compressible de sorte qu'il est susceptible de s'allonger de façon longitudinale in situ d'une première longueur à une seconde longueur plus grande, **caractérisé en ce que** le premier élément annulaire (12) est en forme d'entonnoir et a un plus grand diamètre au niveau d'une extrémité proximale (12a) dans lequel le diamètre dudit premier élément annulaire (12) diminue vers une partie distale (12b).

2. Gaine (10) selon la revendication 1, dans laquelle les premier (12) et second (16) éléments annulaires sont remplis d'au moins un fluide ou une mousse.

3. Gaine (10) selon la revendication 2, dans laquelle la mousse est une mousse sensible à la pression.

4. Gaine (10) selon la revendication 2, dans laquelle la mousse est une mousse thermosensible.

5. Gaine (10) selon la revendication 2, dans laquelle le fluide est un fluide sensible à la pression.

6. Gaine (10) selon la revendication 2, dans laquelle le fluide est un fluide thermosensible.

7. Gaine (10) selon la revendication 1, dans laquelle les premier (12) et second (16) éléments annulaires s'étendent de manière circonférentielle autour d'une périphérie du manchon (14).

8. Gaine (10) selon la revendication 1, dans laquelle les premier (12) et second (16) éléments annulaires comprennent des matières biodégradables.

9. Gaine (10) selon la revendication 1, comprenant en outre un agent bioactif.

10. Gaine (10) selon la revendication 1, dans lequel le manchon (14) inclut en outre un élément de saisie (40) au niveau de l'extrémité proximale (13) pour faciliter l'allongement du manchon.

11. Gaine (10) selon l'une quelconque des revendications précédentes, dans lequel au moins un élément annulaire (12, 16) est un premier ballon et au moins un élément annulaire est un second ballon.
